# EUROPEAN PATENT APPLICATION

(11) **EP 4 368 710 A1**
(43) Date of publication of application: **15.05.2024**
(21) Application number: 22760645.6
(22) Date of filing: 07.07.2022
(51) Int. Cl.: C12N 9/12, A61K 38/00, A61P 35/00, C07K 7/06

(54) **LINEAR PEPTIDES INHIBITING CK2-MEDIATED PHOSPHORYLATION AND COMPOSITIONS COMPRISING SAME**

(30) Priority: 09.07.2021 CU 20210058
(71) Applicant: Centro de Ingeniería Genética y Biotecnología, La Habana 11600 (CU)
(72) Inventor: MASFORROL GONZÁLEZ, Yordanka, La Habana 10600 (CU); GARAY PÉREZ, Hilda, Elisa, La Habana 11600 (CU); REYES ACOSTA, Osvaldo, La Habana 11600 (CU); PERERA NEGRIN, Yasser, La Habana 11600 (CU); CABALLERO MENÉNDEZ, Evelin, La Habana 11600 (CU); GONZÁLEZ LÓPEZ, Luis, Javier, La Habana 11600 (CU); BESADA PÉREZ, Vladimir, Armando, La Habana 11600 (CU); PEREA RODRÍGUEZ, Silvio, Ernesto, La Habana 11600 (CU); GUILLÉN NIETO, Gerardo, Enrique, La Habana 11600 (CU); GONZALEZ BLANCO, Sonia, La Habana 11600 (CU)
(74) Representative: Capitán García, Maria Nuria
(86) International application number: PCT/CU2022/050007
(87) International publication number: WO 2023/280330

(57) **Abstract**

Linear peptides that inhibit phosphorylation mediated by the enzyme casein kinase 2 (CK2), which have an amino acid sequence selected from the group consisting of the sequences identified as SEQ ID NO: 45, SEQ ID NO: 49, SEQ ID NO: 50, and SEQ ID NO: 57 - SEQ ID NO: 62, as well as polypeptides comprising said peptides and an intracellular penetrating peptide. A pharmaceutical composition comprising at least one of these linear peptides or polypeptides and a pharmaceutically acceptable excipient. The invention also discloses the use of said peptides or polypeptides for the manufacture of a drug, and a method of treating solid or liquid tumors where said drug is administered.

## Description

### Technique field

The present invention relates to the field of molecular pharmacology and human medicine. In particular, the invention relates to obtaining linear peptides that inhibit the phosphorylation event mediated by the enzyme Casein Kinase 2 (CK2), through direct interaction with the phosphoacceptor site on the substrate. Due to their biological activity, the peptides and polypeptides of the invention are useful for the treatment of different types of solid tumors and of other origins.

### Previous art

CK2 is a serine/threonine enzyme that is involved in increasing cell proliferation, and its intracellular localization is fundamentally nuclear during the process of malignant transformation (Tawfic S., et al., 2001, Histol. Histopathol. 16:573-582). Therefore, this enzyme constitutes an attractive therapeutic target. (Duncan J.S., et al., 2008, Biochim. Biophys. Acta, 1784: 33-47).

The CK2 enzyme-mediated phosphorylation event has been manipulated in experimental oncology by using chemical compounds derived from 4,5,6,7-tetrabromobenzotriazole (TBB) (Pagano M.A., et al., 2004, J. Med. Chem., 47: 6239-6247), antisense oligonucleotides (Slaton J.W., et al., 2004, Mol. Cancer Res. 2:712-720) and peptides that block the interaction between the catalytic and regulatory subunits of the enzyme (Laudet B., et al., 2007, Biochem. J., 408: 363-373). The chemical compound CX-4945 inhibits the two catalytic subunits of the enzyme CK2 (Siddiqui-Jain A., et al., 2010, Cancer Res. 70: 10288-10298), and is a potent inhibitor of cell proliferation in vitro, with a mean maximum inhibitory concentration (IC₅₀) of 5.5 µM. Only this CK2 enzyme inhibitor has been satisfactorily evaluated in humans, where favorable pharmacokinetic and safety parameters were recorded in Phase I clinical studies (Lim J.K.C., et al., 2010, Cancer Res. 70: 2763-2766 Only this CK2 enzyme inhibitor has been satisfactorily evaluated in humans, where favorable pharmacokinetic and safety parameters were recorded in Phase I clinical studies. (Patent request WO03/054002; Perea S.E., et al., 2004, Cancer Res., 64: 7127-7129). The CIGB-300 peptide is a chimeric molecule that contains a cyclic peptide that interacts with the CK2 phosphoacceptor site on the substrate and an "intracellular penetration" peptide from the Tat1 protein of the Human Immunodeficiency Virus (HIV), to guarantee its internalization in the cell. CIGB-300 has demonstrated antiproliferative and proapoptotic properties, *in vitro* and *in vivo,* in various lines of solid tumors such as lung, cervix, larynx (Perera Y., et al., 2014, Mol Clin Oncol, 2 (6): 935-944) and in liquid tumors such as chronic lymphocytic leukemia (Martins L., et al., 2013, Oncotarget, 5(1): 258-263) and T-type acute lymphoblastic leukemia (Perera Y., et al., 2020, Cancers, 12, 1377). In addition, this peptide showed signs of clinical benefit, verified in Phase I/II clinical studies (Perea S., et al., 2011, Mol Cell Biochem., 356 (1-2): 45-50) (Sarduy M., et al., 2015, Br. J. Cancer, 112: 1636-1643). However, CIGB-300 is a 25 amino acid peptide, containing methionine in its sequence and an intramolecular disulfide bond. These are sources of chemical instability in the molecule, and can be the cause of inconsistencies in the higher stages of product development, whether in the liquid or lyophilized formulation and in stability studies. (Frokjaer S. et al., 2000, Pharmaceutical formulation development of peptides and proteins. Taylor & Francis). The main impurities present in this peptide are due to the oxidation of methionine and the formation of aggregates (dimers and trimers), due to the formation of intermolecular disulfide bonds. (Garay H., et al., 2018, J Pept Sci. 24(6): e3081). On the other hand, the synthesis processes of peptides containing cysteines are more cumbersome. (Albericio F., et al., 2000, Fmoc solid phase peptide synthesis. A Practical Approach. Oxford University Press Inc, New York. Chapter 4, 77-114), and the formation of the intramolecular disulfide bond introduces an extra step in the production processes, which increases production costs. Therefore, it is still of interest to obtain inhibitors of phosphorylation mediated by the CK2 enzyme, of the linear peptide type, of smaller size and structural complexity, whose synthesis is simpler and cheaper, for the development of therapies against different types of tumors.

### Description of the invention

The present invention solves the aforementioned problem by providing linear peptides that inhibit the CK2-mediated phosphorylation and that have an amino acid sequence that is selected from the group consisting of the sequences identified as SEQ ID NO: 45, SEQ ID NO: 49, SEQ ID NO: 50, and SEQ ID NO: 57 - SEQ ID NO: 62. These sequences are shown below:

| | | |
|---|---|---|
| a) | SEQ ID NO: 45 | RKRSRYWP |
| b) | SEQ ID NO: 49 | RKRWRYWP |
| c) | SEQ ID NO: 50 | RKRSWYWP |

| | | |
|---|---|---|
| d) | SEQ ID NO: 57 | R(D-Lys)RWRYWP |
| e) | SEQ ID NO: 58 | RK(D-Arg)WRYWP |
| f) | SEQ ID NO: 59 | RKRWR(NMe-Tyr)WP |
| g) | SEQ ID NO: 60 | R(D-Lys)RSWYWP |
| h) | SEQ ID NO: 61 | RK(D-Arg)SWYWP |
| i) | SEQ ID NO: 62 | RKRSW(NMe-Tyr)WP |

These peptides differ from CIGB-300 in that they are linear, smaller in size, and chemically more stable. They do not have methionine or cysteine in their sequence, which is an advantage. In addition, from the productive point of view, the obtention is more feasible.

The linear peptides identified as SEQ ID NO: 45, SEQ ID NO: 49, SEQ ID NO: 50, and SEQ ID NO: 57 - SEQ ID NO: 62 were selected for their ability to interact with the phosphoacceptor site of the CK2 enzyme on the E7 substrate of the Human Papillomavirus (HPV)-16 (LNDSSEEEDEI), and also for its ability to inhibit CK2-mediated phosphorylation of this oncoprotein. As seen in the examples of the present invention, these linear peptides are capable of inhibiting CK2-mediated phosphorylation of the E7 protein in a similar manner to peptide CIGB-300.

To achieve intracellular action on endogenous CK2 substrates, the peptides of the invention can be chemically conjugated with peptides of "intracellular penetration", belonging to proteins such as the Tat1 protein of HIV (Schwarze S.R., et al., 2000, Trends Pharmacol, 21: 45-48), la proteína VP22 del Virus Herpes Simples (Lindgreen M., et al., 2000, Trends Pharmacol Sci, 21: 99-103), the *Penetratin* and the *Transportan* (Gariepy J., et al., 2001, Trends Biotech 19: 21-28), among others. Therefore, the invention also provides polypeptides comprising linear peptides that have an amino acid sequence that is selected from the sequences identified as SEQ ID NO: 45, SEQ ID NO: 49, SEQ ID NO: 50, and SEQ ID NO: 57 - SEQ ID NO: 62 and one cell penetrating peptide.

In one embodiment of the invention, the intracellularly penetrating peptide in these polypeptides is a peptide from the Tat1 protein of HIV. In one embodiment thereof, the intracellularly penetrating peptide has an amino acid sequence that is identified as SEQ ID NO: 65 either SEQ ID NO: 66.

In a particular embodiment, the polypeptide of the invention has an amino acid sequence that is selected from the group consisting of the sequences identified as SEQ ID NO: 35, SEQ ID NO: 47, SEQ ID NO: 48, and SEQ ID NO: 51 - SEQ ID NO: 56. These sequences are shown below:

| | | |
|---|---|---|
| a) | SEQ ID NO: 35 | GRKKRRQRRRPPQ-βA-RKRSRYWP |
| b) | SEQ ID NO: 47 | GRKKRRQRRRPPQ-βA-RKRWRYWP |
| c) | SEQ ID NO: 48 | GRKKRRQRRRPPQ-βA-RKRSWYWP |
| d) | SEQ ID NO: 51 | Ac-GRKKRRQ(D-Arg)RRPPQ-βA-R(D-Lys)RWRYWP |
| e) | SEQ ID NO: 52 | Ac-GRKKRRQ(D-Arg)RRPPQ-βA-RK(D-Arg)WRYWP |
| f) | SEQ ID NO: 53 | Ac-GRKKRRQ(D-Arg)RRPPQ-βA-RKRWR(NMe-Tyr)WP |
| g) | SEQ ID NO: 54 | Ac-GRKKRRQ(D-Arg)RRPPQ-βA-R(D-Lys)RSWYWP |
| h) | SEQ ID NO: 55 | Ac-GRKKRRQ(D-Arg)RRPPQ-βA-RK(D-Arg)SWYWP |
| i) | SEQ ID NO: 56 | Ac-GRKKRRQ(D-Arg)RRPPQ-βA-RKRSW(NMe-Tyr)WP |

Most of these polypeptides are more active than peptide CIGB-300, as they have a lower IC₅₀ in various cancer cell lines (lung, bladder, larynx, pancreas, and leukemia). The polypeptides were able to produce a dose-dependent effect on the proliferation of cancer cells. Besides, the polypeptide identified as SEQ ID NO: 35 shows IC₅₀ values similar to those of CIGB-300 in these cell lines. However, it is considered superior because it has a linear amino acid sequence, smaller in size and more chemically stable, with productive advantages that accelerate the development of the therapeutic product.

For the identification of the peptides described in the invention, a combinatorial library of linear peptides by type "one-bead-one-compound" was synthesized, containing more than 16 million of different peptide sequences, and the screening was carried out with a synthetic peptide comprising the 28-38 region of the E7 oncoprotein modified with Biotin. With the use of mass spectrometry, the sequences of the peptides contained in the nine beads that were positive to the screening were assigned. The peptides identified a SEQ ID NO: 1- SEQ ID NO: 9 were chemically synthesized, and it was confirmed that they were capable of inhibiting CK2-mediated phosphorylation in the E7 oncoprotein of HPV-16. In order to achieve intracellular action on endogenous CK2 substrates, polypeptides containing the nine peptides were designed and synthesized, in which the intracellular penetration peptide corresponding to the HIV Tat1 protein was added at the N-terminus (GRKKRRQRRRPPQ, identified as SEQ ID NO: 65). Next, the effect of these nine polypeptides on cell growth of the NCI-H125 line (non-small cell lung cancer) was evaluated. None of the polypeptides reached inhibition values similar to CIGB-300, in the range of the concentrations evaluated. Only three of them, identified as SEQ ID NO: 18 - SEQ ID NO: 20, inhibited between 20% and 30% cell proliferation at the highest concentration studied, so modifications were made to their sequences to improve their biological activity.

A Trp positional scanning library was designed and obtained to assess the impact of Trp incorporation on the biological activity of polypeptides identified as SEQ ID NO: 18 - SEQ ID NO: 20. Next, the antiproliferative effect of the 24 polypeptides generated in the Trp library (identified as SEQ ID NO: 21 - SEQ ID NO: 44) in the NCI-H125 cell line. Six of the 24 polypeptides, identified as SEQ ID NO: 21, SEQ ID NO: 25, SEQ ID NO: 27, SEQ ID NO: 32, SEQ ID NO: 35 and SEQ ID NO: 43, showed percentages of inhibition of cell proliferation similar to peptide CIGB-300 at the highest concentration evaluated. The IC₅₀ values of the polypeptide identified as SEQ ID NO: 35 and CIGB-300, obtained in the NCI-H125 and AsPC-1 (pancreatic adenocarcinoma) cell lines are comparable. Particularly, the polypeptide identified as SEQ ID NO: 35 is considered superior to CIGB-300, because it is a linear sequence of smaller size, more chemically stable and with productive advantages.

Two other linear polypeptides more active than the CIGB-300 peptide, identified in the invention as SEQ ID NO: 47 and SEQ ID NO: 48, are the result of a study where a second Trp residue was introduced at three positions in the polypeptide identified as SEQ ID NO: 35. It was shown that the polypeptides identified as SEQ ID NO: 47 and SEQ ID NO: 48 are more potent than peptide CIGB-300, in their ability to inhibit cell proliferation in pancreatic cancer (AsPC-1, PanC-1) and acute myeloid leukemia (OCI-AML3) cell lines, and were similar to CIGB- 300 in the lung cancer cell line used (NCI-H 125).

As a result of the introduction of unnatural amino acids in the sequences identified as SEQ ID NO: 47 and SEQ ID NO: 48, in sites prone to enzymatic degradation, six new linear polypeptides more active than CIGB-300 were obtained, which were identified as SEQ ID NO: 51 - SEQ ID NO: 56. Antiproliferative assays were performed on the NCI-H125, Hep2C (laryngeal carcinoma) cell lines, and on two bladder cancer lines (MGH-U3 and MGH-U4). The IC₅₀ values of the peptides identified as SEQ ID NO: 51 - SEQ ID NO: 56, obtained in the NCI-H125, Hep2C, MGH-U3 and MGH-U4 cell lines, are lower than those obtained for the CIGB-300, which shows that they are more potent. In the case of the bladder cancer lines (MGH-U3 and MGH-U4), the IC₅₀ values are lower by an order of magnitude compared to the CIGB-300, which is very attractive as it is a little explored niche, not resolved and with a high impact on health. The introduction of the unnatural amino acids was done to increase the stability to proteases. Surprisingly, it was also possible to enhance the ability of these polypeptides to inhibit CK2-mediated phosphorylation.

The invention also provides pharmaceutical compositions that comprise at least one linear peptide that has an amino acid sequence that is selected from the group consisting of the sequences identified as SEQ ID NO: 45, SEQ ID NO: 49, SEQ ID NO: 50, and SEQ ID NO: 57 - SEQ ID NO: 62, or at least one polypeptide that has an amino acid sequence that is selected from the group consisting of the sequences identified as SEQ ID NO: 35, SEQ ID NO: 47, SEQ ID NO: 48, and SEQ ID NO: 51 - SEQ ID NO: 56, and a pharmaceutically acceptable excipient.

The excipients that can form part of the pharmaceutical compositions of the invention are known to those skilled in this field of the art. In one embodiment of the invention, the pharmaceutical composition is formulated for administration by the systemic, intratumoral, oral, or mucosal route. In one embodiment of the invention, the pharmaceutical compositions are useful for the treatment of solid or liquid tumors in an individual in need thereof.

The object of the invention is the use of linear peptides that have a sequence that is selected from the group composed of the sequences identified as SEQ ID NO: 45, SEQ ID NO: 49, SEQ ID NO: 50, and SEQ ID NO: 57 - SEQ ID NO: 62, or polypeptides that have an amino acid sequence that is selected from the group consisting of the sequences identified as SEQ ID NO: 35, SEQ ID NO: 47, SEQ ID NO: 48, and SEQ ID NO: 51 - SEQ ID NO: 56, to manufacture a drug.

In an embodiment of the invention, said medicament is used in the treatment of solid or liquid tumors.

In another aspect, the invention discloses a method of treating solid or liquid tumors in an individual in need thereof comprising administering a therapeutically effective amount of a pharmaceutical composition comprising at least one linear peptide having an amino acid sequence that is selected from the group composed of the sequences identified a SEQ ID NO: 45, SEQ ID NO: 49, SEQ ID NO: 50, and SEQ ID NO: 57 - SEQ ID NO: 62, or at least one polypeptide that has an amino acid sequence that is selected from the group consisting of the sequences identified as SEQ ID NO: 35, SEQ ID NO: 47, SEQ ID NO: 48, and SEQ ID NO: 51 - SEQ ID NO: 56. In one embodiment of the invention, the solid tumor being treated is a tumor of the lung, cervix, larynx, pancreas, or bladder. In another embodiment of the invention, the liquid tumor being treated is chronic lymphocytic leukemia, T-type acute lymphoblastic leukemia, or acute myeloid leukemia.

### Brief description of the figures

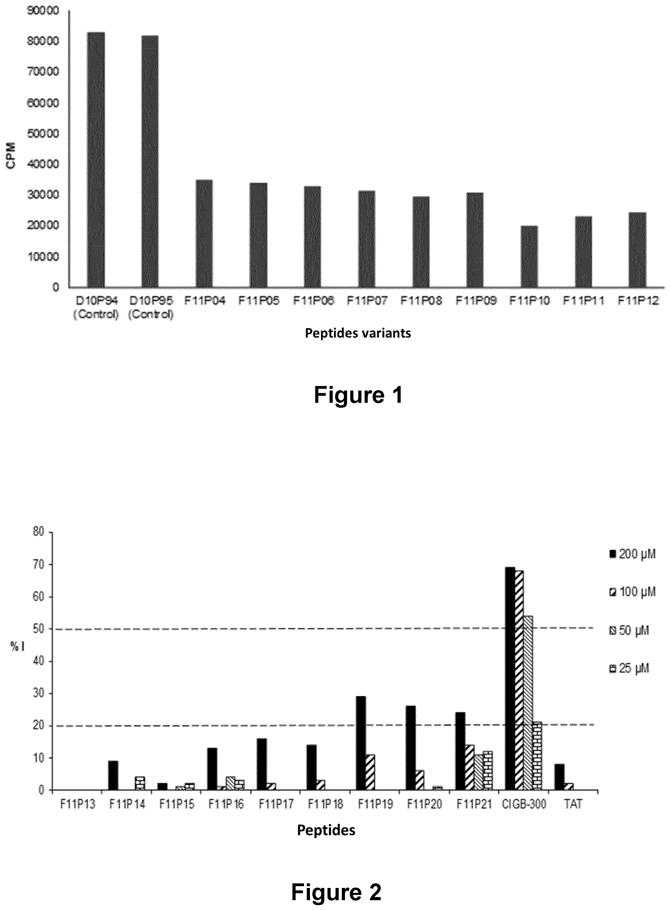

**Figure 1****.** Effect of synthetic peptides on SEQ ID NO: 1 - SEQ ID NO: 9 on the CK2-mediated phosphorylation of the HPV-16E7 oncoprotein. Two peptides that do not interact with the phosphoacceptor site of the CK2 enzyme (identified as SEQ ID NO: 10 and SEQ ID NO: 11). Counts per minute (cpm) are plotted on the Y-axis.
**Figure 2****.** Percentage inhibition of cell proliferation (%I) in the NCI-H125 cell line for the nine polypeptides identified as SEQ ID NO: 12 - SEQ ID NO: 20. Peptides CIGB-300 and Tat were used as positive and negative control of the assay, respectively. Polypeptides showing a percentage inhibition of cell proliferation ≥ 20% were selected.

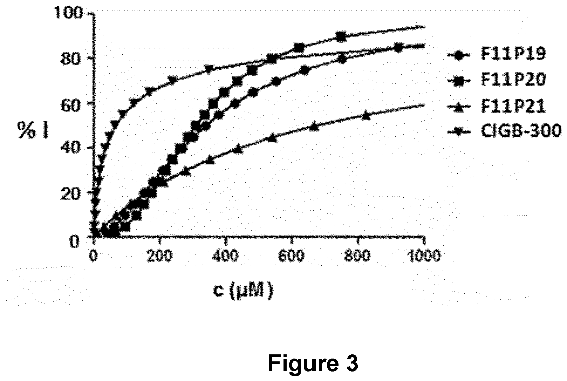

**Figure 3****.** Dose-effect curves of polypeptides F11P19, F11P20 and F11P21 (SEQ ID NO: 18 - SEQ ID NO: 20) in the NCI-H125 cell line. The dose-effect curve of the CIGB-300 peptide that was used as a positive control of the assay is included. % I: Percentage of inhibition of cell proliferation. C: concentration.

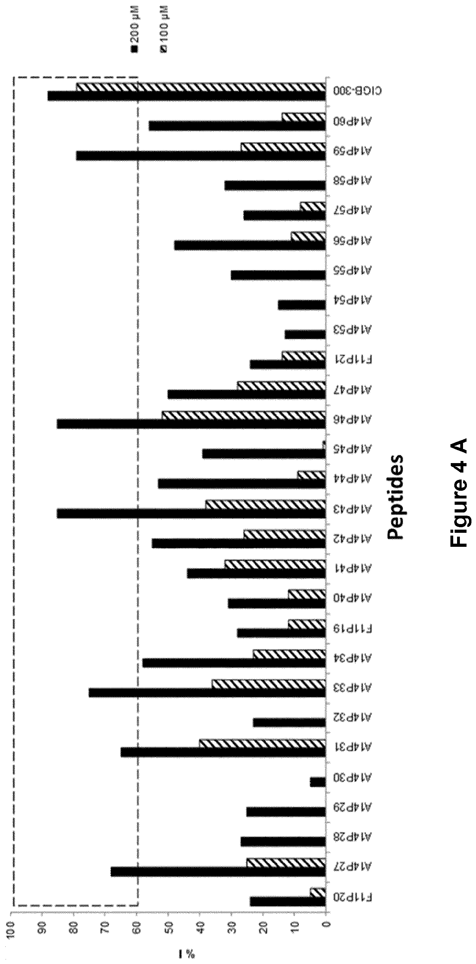

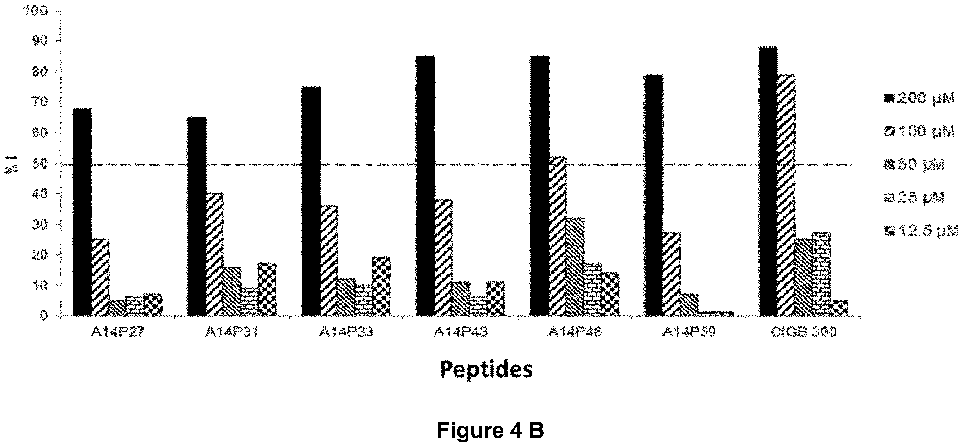

**Figure 4****.** Percentage of inhibition of cell proliferation (% I) in the NCI-H125 cell line for the polypeptides generated from the sequences identified as SEQ ID NO: 18 - SEQ ID NO: 20, by positional sweep of the Trp residue in the charge (SEQ ID NO: 21 - SEQ ID NO: 44). **A.** Percentage of inhibition achieved by the polypeptides identified as SEQ ID NO: 21 - SEQ ID NO: 44, at two concentrations. The precursor peptides identified as SEQ ID NO: 18 - SEQ ID NO: 20 and CIGB-300 as a positive control. The dashed box outlines the six polypeptides that showed greater than 60% inhibition of cell proliferation at 200 µM and the CIGB-300. **B.** Inhibition percentage achieved by the six most active polypeptides at various concentrations between 12.5 µM and 200 µM.

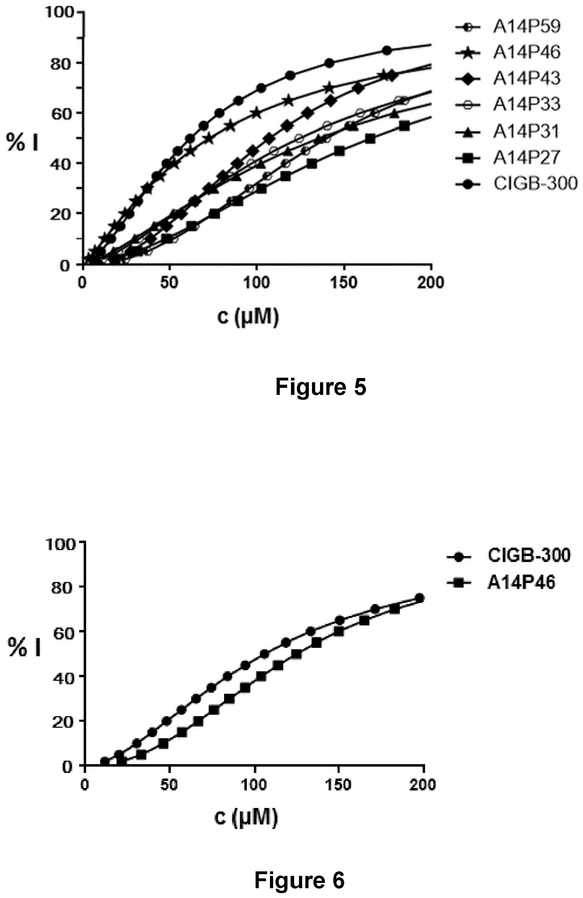

**Figure 5****.** Dose-effect curves of polypeptides A14P27 (SEQ ID NO: 21), A14P31 (SEQ ID NO: 25), A14P33 (SEQ ID NO: 27), A14P43 (SEQ ID NO: 32), A14P46 (SEQ ID NO: 35), A14P59 (SEQ ID NO: 43) in the NCI-H125 cell line. The dose-effect curve of the CIGB-300 peptide used as control peptide is included. % I: Percentage of inhibition of cell proliferation. C: concentration.
**Figure 6****.** Dose-effect curves of polypeptide A14P46 (SEQ ID NO: 35) and the CIGB-300 peptide in the AsPC-1 cell line. % I: Percentage of inhibition of cell proliferation. C: concentration.

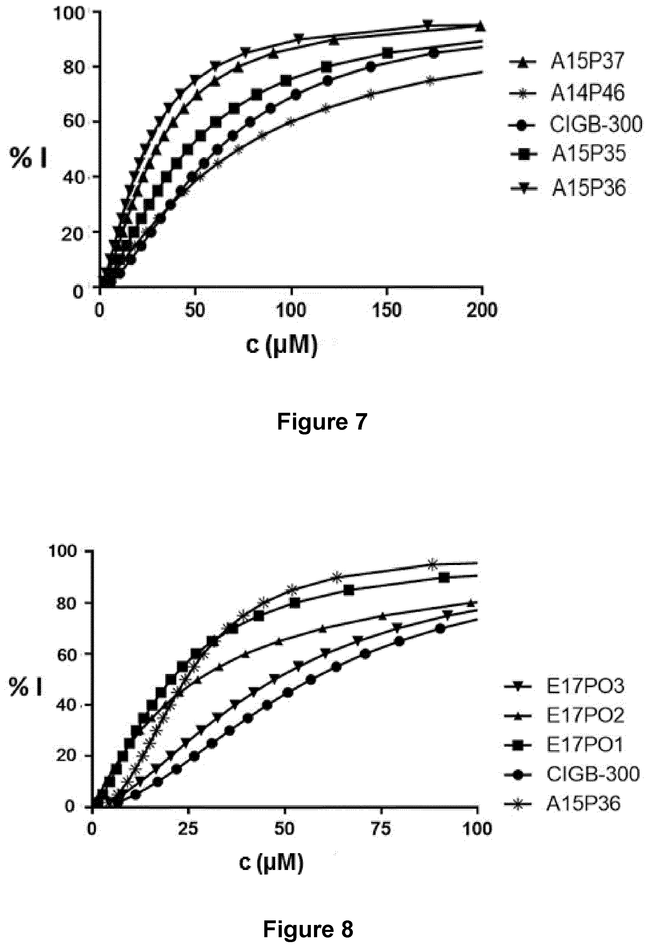

**Figure 7****.** Dose-effect curves of polypeptides A15P35, A15P36 and A15P37 (SEQ ID NO: 46 - SEQ ID NO: 48) in the NCI-H125 cell line. Dose-effect curves of the precursor polypeptide A14P46 are included (SEQ ID NO: 35) and the CIGB-300 peptide that was used as a control. % I: Percentage of inhibition of cell proliferation. C: concentration.
**Figure 8****.** Dose-effect curves of polypeptides E17P01, E17P02 and E17P03 (SEQ ID NO: 51 - SEQ ID NO: 53) in the NCI-H125 cell line. Dose-effect curves of the precursor polypeptide A15P36 are included. (SEQ ID NO: 47) and CIGB-300 that was used as a control. % I: Percentage of inhibition of cell proliferation. C: concentration.

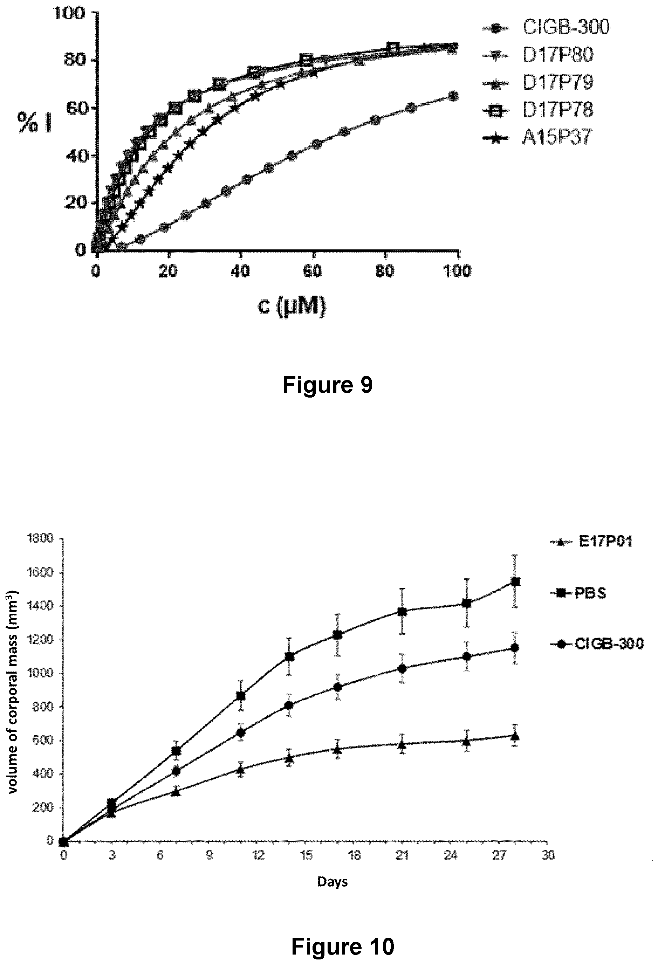

**Figure 9****.** Dose-effect curves of polypeptides D17P78, D17P79 and D17P80 (SEQ ID NO: 54 - SEQ ID NO: 56) in the NCI-H125 cell line. Dose-effect curves of the precursor polypeptide are included A15P37 (SEQ ID NO: 48) and CIGB-300 that was used as a control. % I: Percentage of inhibition of cell proliferation. C: concentration.
**Figure 10****.** Antitumor effect of the polypeptide inhibitor of CK2 phosphorylation E17P01 (SEQ ID NO: 51) in a human bladder tumor model implanted in athymic mice.

### Detailed exposition of embodiments / Examples of embodiment

### Example 1. Selection of linear peptides that interact with the phosphoacceptor site of the CK2 enzyme in the E7 protein of HPV-16.

For the identification of bioactive linear peptides that interact with the phosphoacceptor site of the CK2 enzyme, a combinatorial library of synthetic linear peptides named BCP-1 by type of "one-bead-one-compound" was screened, containing more than 16 million of different peptide sequences. This library was designed to be compatible with electrospray ionization mass spectrometry (ESI-MS) sequence analysis (Masforrol Y., et al., 2012, ACS Combinatorial Science, 14(3): 145-9). Screening was performed with a biotinylated synthetic peptide, called BIOT, which contains the phosphoacceptor site of the CK2 enzyme in the E7 substrate of HPV-16 (LNDSSEEEDEI, identified as SEQ ID NO: 63). The streptavidin-alkaline phosphatase conjugate was used as recognition molecule, and p-toluidine salt of 5-bromo-4-chloro-3-indolyl-phosphate (BCIP) was used as substrate for development. The blue color facilitates the visual differentiation of the beads containing bioactive sequences recognized by the BIOT peptide, compared to the bright light yellow color of the TentaGel resin at the BCP-1 library. The BIOT peptide was synthesized on 4-methylbenzhydrylamine (MBHA) resin, using the Fmoc/tBu chemistry (Field GB and Noble RL. Int. J. Peptide Protein Res. 1990; 35(3):161-214). It was purified by reverse phase chromatography (RP-HPLC) and its identity was confirmed by ESI-MS. It was obtained with more than 95% purity, and ESI-MS analysis confirmed its molecular mass (MM) of 1574.6 Da.

The BCP-1 library was placed in a polypropylene reactor with a vacuum filtration system and mechanical agitation. It was washed with purified water and saline phosphate buffer solution (PBS) pH 7.4; and blocked with 1% bovine serum albumin (BSA) in PBS pH 7.4 for one hour. Solvents and reagents were removed by vacuum filtration. The library was incubated with 80 mL of a BIOT peptide solution at 150 µg/mL in 1% BSA in PBS pH 7.4, for 16 h at 4°C. It was washed with Tween-PBS (T-PBS) pH 7.4; and incubated with the streptavidin-alkaline phosphatase conjugate (0.4 µg/mL) in a solution of 1% BSA in PBS pH 7.4. Two hours later, it was washed with T-PBS pH 7.4 and incubated with BCIP (0.5 mg/mL) in substrate solution for 30 min. When the blue color appeared, the reaction was stopped by filtration and abundant washings with PBS pH 7.4.

The positive beads were separated from the rest by visual examination, with the aid of an ocular lens, transferred to a reactor with a frit, and regenerated. Subsequently, tests were performed to rule out nonspecific recognition by the streptavidin-alkaline phosphatase conjugate and BCIP. Screening was carried out under conditions of greater astringency, to select the sequences of greater affinity and reduce the number of positive beads, for which the concentration of the BIOT peptide was reduced to 120 µg/mL. Positive beads were regenerated and sequence identification was carried out by nanoESI-MS/MS, according to the procedure described above (Masforrol Y., et al, 2012, ACS Combinatorial Science, 14(3): 145-9).

As a result of the initial screen with the peptide BIOT at 150 µg/mL, 56 positive beads were selected. After the second screening with a lower concentration of BIOT (120 µg/mL), nine positive beads were selected that showed a more intense blue coloration than the rest of the positive beads at the initial screening, which evidenced the greater affinity of these peptides for the CK2 phosphoacceptor site. The nanoESI-MS/MS spectra obtained from the nine positive beads were analyzed in parallel, by manual de novo sequencing and automated sequencing, using the Mascot program to search the BCP-1 databases. Table 1 shows the sequence of the nine peptides identified (SEQ ID NO: 1 - SEQ ID NO: 9). From the analysis of the sequences assigned to each peptide, a complete correspondence of the assignment of the m/z signals between the manual identification and the automatic sequencing is obtained, which allowed the accurate identification of the peptide sequences in each case.

**Table 1. Amino acid sequence of the peptides identified in the nine positive beads isolated in the screening of the BCP-1 library with the BIOT peptide.**

| **Bead** | **Peptide sequence in the bead** | **SEQ ID. No** |
|---|---|---|
| 1 | RLRRKADP | 1 |
| 2 | LRRRKADP | 2 |
| 3 | TYRYRRDP | 3 |
| 4 | VARRRYDP | 4 |
| 5 | AVRRRYDP | 5 |
| 6 | TFYRRYDP | 6 |
| 7 | RKRSRYDP | 7 |
| 8 | GYRYKRDP | 8 |
| 9 | VKKRRLDP | 9 |

A similarity was observed in the amino acid composition of the nine peptides shown in Table 1. This is characterized by the predominance of basic amino acids (K, R) and the presence of hydrophobic amino acids (L, A, V, F, Y).

To corroborate that the nine sequences identified by nanoESI-MS/MS interact with the phosphoacceptor site of the CK2 enzyme in the E7 protein, the peptides were synthesized directly on the Tentagel resin, using the Fmoc/tBu chemistry (Field G.B. et al., 1990, Int. J. Peptide Protein Res., 35(3):161-214). An enzymatic reaction was carried out on the peptide coupled to the resin, with the BIOT peptide at a concentration of 120 µg/mL The streptavidin-alkaline phosphatase conjugate was used as recognition molecule, and BCIP was used as substrate for development. Nine reactors containing 15 mg of the encoded peptides (P1-P9) coupled to resin were placed in a vacuum filtration system, with mechanical agitation. Two control peptides coupled to the resin were used (P10 identified as SEQ ID NO: 10 and P11 identified as SEQ ID NO: 11) that do not interact with the CK2 phosphoacceptor site on the E7 protein. The procedure was adjusted to reproduce the BCP-1 library screening assay in a 500 µL working volume for each reactor. The results showed that the nine peptides coupled to the resin (P1-P9) developed an intense blue color after enzymatic assay, which evidences the ability of the nine identified linear peptides to interact with the phosphoacceptor site of the CK2 enzyme in the substrate. E7 model. In contrast, the resin-coupled control peptides (P10 and P11) did not develop blue coloration.

### Example 2. Effect of the peptides identified as SEQ ID NO: 1 - SEQ ID NO: 9 on the CK2-mediated phosphorylation of the HPV-16 E7 oncoprotein.

Peptides were synthesized on MBHA resin using Fmoc/tBu chemistry (Field GB and Noble RL. Int. J. Peptide Protein Res. 1990; 35(3):161-214). Peptides were purified by RP-HPLC and their identity confirmed by ESI-MS. For synthesis, they were designated as F11P04 - F11P12, and their amino acid sequences correspond to the sequences identified as SEQ ID NO: 1- SEQ ID NO: 9. As seen in Table 2, all were obtained with more than 95% purity and ESI-MS analysis confirmed their identity.

**Table 2. Characterization of the synthetic peptides containing the sequences identified after screening the BCP-1 library with BIOT.**

| **SEQ ID NO:** | **Synthesis code** | **Sequence** | **Purity (%)** | **MW (Da)** |
|---|---|---|---|---|
| 1 | F11P04 | RLRRKADP | 99.1 | 1009.6 |
| 2 | F11P05 | LRRRKADP | 99.1 | 1009.6 |
| 3 | F11P06 | TYRYRRDP | 98.1 | 1124.6 |
| 4 | F11P07 | VARRRYDP | 99.3 | 1030.6 |
| 5 | F11P08 | AVRRRYDP | 98.6 | 1030.6 |
| 6 | F11P09 | TFYRRYDP | 98.9 | 1115.5 |
| 7 | F11P10 | RKRSRYDP | 98.8 | 1075.7 |
| 8 | F11P11 | GYRYKRDP | 97.9 | 1052.5 |
| 9 | F11P12 | VKKRRLDP | 99.6 | 1009.7 |
| 10 | D10P94 | EQPAFHDP | 98.5 | 938.6 |
| 11 | D10P95 | VAGFFLDP | 98.7 | 863.4 |

The assay to measure the effect of these peptides on the CK2-mediated phosphorylation of the HPV-16 E7 oncoprotein, was based on an in vitro phosphorylation reaction, where the E7 oncoprotein of HPV-16 expressed in *Escherichia coli* was used as substratum. This oncoprotein was obtained as a fusion protein to Glutathione S-Transferase. The assay was performed according to the procedure previously described. (Perea S.E., et al., 2004, Cancer Res., 64: 7127-7129). The results are shown in Figure 1, and indicate that the peptides identified as SEQ ID NO: 1- SEQ ID NO: 9 are capable of inhibiting CK2-mediated phosphorylation of the E7 protein of HPV-16.

### Example 3. Effect of synthetic polypeptides identified as SEQ ID NO: 12 - SEQ ID NO: 20 on cell growth in the non-small cell lung cancer line NCI-H125.

In order to achieve intracellular action on endogenous CK2 substrates, the sequences identified as SEQ ID NO: 1 - SEQ ID NO: 9 the intracellular penetration peptide Tat, corresponding to the Tat1 protein of HIV, was added at the N-terminus, (GRKKRRQRRRPPQ). A beta Alanine (βA) residue was introduced as a spacer between the peptide of interest and the Tat peptide. The polypeptides were coded as F11P13- F11P21 for synthesis, and correspond to the sequences identified as SEQ ID NO: 12 - SEQ ID NO: 20, as shown in Table 3. Peptide CIGB-300 was identified as SEQ ID NO: 64, and the Tat peptide was identified as SEQ ID NO: 65. The synthesis, purification and identity analysis of the peptides was carried out as shown in Example 2. As shown in Table 3, all of them were obtained with more than 95% purity and the ESI-MS analysis confirmed their identity.

**Table 3. Characterization of the synthetic polypeptides identified as SEQ ID NO: 12 - SEQ ID NO: 20.**

| **SEQ ID NO:** | **Synthesis code** | **Sequence** | **Purity (%)** | **MW (Da)** |
|---|---|---|---|---|
| 12 | F11P13 | GRKKRRQRRRPPQ-βA-RLRRKADP | 98.7 | 2780.8 |
| 13 | F11P14 | GRKKRRQRRRPPQ-βA-LRRRKADP | 98.7 | 2780.7 |
| 14 | F11P15 | GRKKRRQRRRPPQ-βA-TYRYRRDP | 96.6 | 2895.8 |
| 15 | F11P16 | GRKKRRQRRRPPQ-βA-VARRRYDP | 97.2 | 2801.7 |
| 16 | F11P17 | GRKKRRQRRRPPQ-βA-AVRRRYDP | 98.2 | 2801.7 |
| 17 | F11P18 | GRKKRRQRRRPPQ-βA-TFYRRYDP | 96.9 | 2886.8 |
| 18 | F11P19 | GRKKRRQRRRPPQ-βA-RKRSRYDP | 96.6 | 2846.7 |
| 19 | F11P20 | GRKKRRQRRRPPQ-βA-GYRYKRDP | 97.4 | 2823.7 |
| 20 | F11P21 | GRKKRRQRRRPPQ-βA-VKKRRLDP | 97.4 | 2780.7 |
| 64 | CIGB-300 | cycle (Cys15-Cys 25) GRKKRRQRRRPPQ- βA-CWMSPRHLGTC | 99.2 | 3057.6 |
| 65 | Tat | GRKKRRQRRRPPQ | 98.3 | 1717.1 |

The evaluation of the antiproliferative activity of polypeptides F11P13-F11P21 (SEQ ID NO: 12 - SEQ ID NO: 20) was performed on the NCI-H125 cell line. The cell line exhibits high sensitivity to low concentrations of the drug under evaluation (HU L.-Y., et al, 2018, European Review for Medical and Pharmacological Sciences, 22: 4551-4556). The antiproliferative effect of the CIGB-300 peptide has been widely characterized in this line (Cirigliano S.M., et al., 2017, Cancer Cell International, 17, 42), therefore, it was selected as a positive control. Tat peptide was used as a negative control. The polypeptides were evaluated at the concentrations of 25, 50, 100 and 200 µM. The cell proliferation assay was performed by a previously described procedure (Perera Y., et al., 2012, J. Pept. Sci, 18 (4):215-23).

Figure 2 show data from the antiproliferative assay in NCI-H125 cells. Each bar corresponds to the values of the percentage of inhibition of cell proliferation (% I) reached for the concentrations of the polypeptides evaluated (25, 50, 100 and 200 µM). The polypeptides that showed a cell proliferation inhibition value greater than or equal to 20% were selected, since this percentage value is within the coefficient of variation of the assay. Only the polypeptides F11P19, F11P20 and F11P21 inhibited cell proliferation between 20% and 30%, at the concentration of 200 µM. Only 50% inhibition of proliferation was achieved with peptide CIGB-300, used as a control. Figure 3 shows the dose-effect curves of the polypeptides F11P19, F11P20 and F11P21, in comparison with the peptide CIGB-300. The IC₅₀ values for polypeptides F11P19, F11P20, F11P21 and for CIGB-300 were 336 µM, 305 µM, 668 µM and 64 µM, respectively. The IC₅₀ values of the polypeptides F11P19, F11P20, F11P21 are still far from the IC₅₀ value obtained for CIGB-300, so it is necessary to optimize said primary structures to improve their biological activity.

### Example 4. Optimization of the sequences identified as SEQ ID NO: 18- SEQ ID NO: 20 by means of a Tryptophan scan in charge.

The Trp amino acid was excluded in the BCP-1 library synthesis by design (Masforrol Y., et al, 2012, ACS Combinatorial Science, 14(3): 145-9). For this reason, Trp position scanning libraries were designed for the sequences identified as SEQ ID NO: 18 - SEQ ID NO: 20, in which the punctual substitution of each amino acid residue for the Trp in charge was performed (Table 4). This allowed us to study the contribution of Trp at each position of the cargo sequence.

Trp position-scanning library polypeptides were synthesized on MBHA resin using Fmoc/tBu chemistry (Field GB y Noble RL. Int. J. Peptide Protein Res. 1990; 35(3):161-214), RP-HPLC purified and identity confirmed by ESI-MS. As can be seen in Table 4, which shows the amino acid sequences, all of them were obtained with more than 95% purity and the ESI-MS analysis confirmed the identity with the designed molecule.

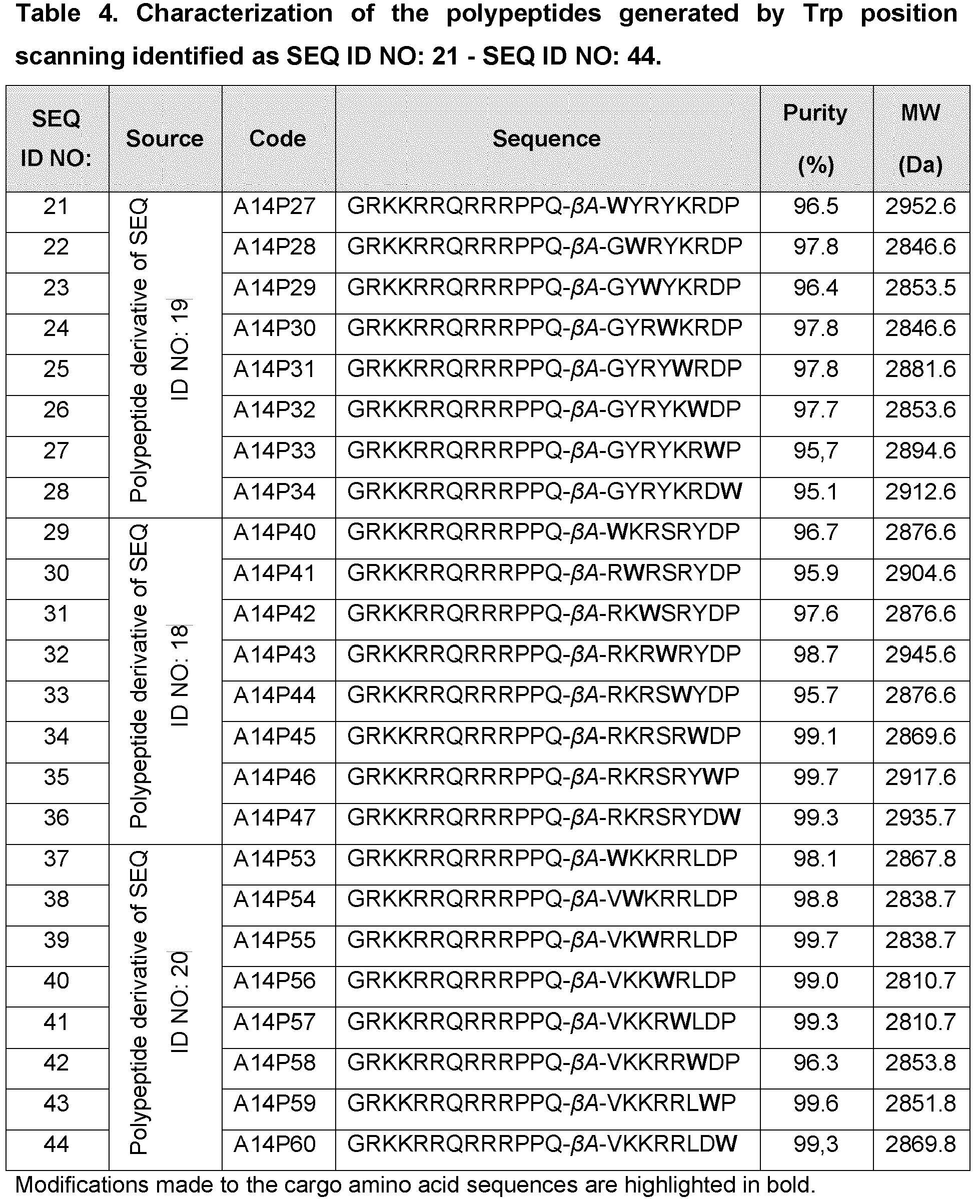

To corroborate that the modification with Trp does not affect the ability of the polypeptides to interact with the phosphoacceptor site of the CK2 enzyme in the E7 protein, the procedure described in Example 1 was followed. The 24 polypeptides coupled to the resin developed a blue color intense after enzyme assay, evidencing interaction with the phosphoacceptor site of the CK2 enzyme in the model E7 substrate. On the other hand, to demonstrate that the modification with Trp does not affect the effect of the polypeptides on the phosphorylation of the E7 protein by CK2, the procedure described in Example 2 was followed. The 24 polypeptides identified as SEQ ID NO: 21- SEQ ID NO: 44 maintained the ability to inhibit the phosphorylation of the E7 protein of HPV-16, mediated by CK2, since phosphorylation inhibition values between 60-85% were obtained. CIGB-300 showed 87% inhibition of phosphorylation. Evaluation of the antiproliferative activity of synthetic polypeptides generated by positional scanning of Trp (SEQ ID NO: 21- SEQ ID NO: 44) was performed on the NCI-H125 cell line. The test was performed by the procedure previously described. (Perera Y., et al., 2012, J. Pept. Sci, 18(4): 215-23). Precursor polypeptides (F11P19, F11P20, F11P21) and peptide CIGB-300 were included as positive controls. The polypeptides were evaluated at the concentrations of 12.5; 25; fifty; 100 and 200 µM. Figure 4A shows the results of percent inhibition of cell proliferation for polypeptide concentrations of 100 and 200 µM. The results show that six of the 24 polypeptides evaluated (A14P27 (SEQ ID NO: 21), A14P31 (SEQ ID NO: 25), A14P33 (SEQ ID NO: 27), A14P43 (SEQ ID NO: 32), A14P46 (SEQ ID NO: 35), A14P59 (SEQ ID NO: 43)) showed more than 60% inhibition of cell proliferation at 200 µM, similar to the effect found with peptide CIGB-300. Figure 4B shows the percentage values of inhibition of cell proliferation obtained for the six selected polypeptides, at the concentrations evaluated. As can be seen, the A14P46 polypeptide inhibits more than 50% of cell proliferation at concentrations above 100 and 200 µM, similar to the behavior of CIGB-300. At the lower concentrations, the A14P46 polypeptide showed a better dose-effect correlation compared to the performance of the CIGB-300 peptide.

Figure 5 shows the dose-effect curves of the polypeptides A14P27, A14P31, A14P33, A14P43, A14P46, A14P59 and CIGB-300. For its part, Table 5 shows that the IC₅₀ values for these six polypeptides are lower than those obtained for their precursor polypeptides F11P19 (336 µM), F11P20 (305 µM) and F11P21 (668 µM). The IC₅₀ of the A14P46 polypeptide (72 µM) is comparable to the IC₅₀ of the CIGB-300 reference peptide (64 µM).

The structural study performed on the peptide sequence X₁X₂X₃X₄X₅X₆X₇X₈, allowed to conclude that the change of D by W in position X₇ is essential to enhance the biological activity (A14P46 >> A14P33 > A14P59), with the best response for the polypeptide A14P46. Additionally, it was concluded that the positions X4 > X5 > X1 also showed remarkable results regarding biological activity, which suggested the introduction of a second amino acid W in substitution of said positions.

**Table 5. IC₅₀ values of polypeptides identified as SEQ ID NO: 21, SEQ ID NO: 25, SEQ ID NO: 27, SEQ ID NO: 32, SEQ ID NO: 35 and SEQ ID NO: 43 in the cell proliferation assay with the NCI-H125 line.**

| **SEQ ID NO:** | **Code** | **Sequence** | **IC₅₀ (µM)** |
|---|---|---|---|
| 21 | A14P27 | GRKKRRQRRRPPQ-*βA*-**W**YRYKRDP | 165 |
| 25 | A14P31 | GRKKRRQRRRPPQ-*βA*-GYRY**W**RDP | 135 |
| 27 | A14P33 | GRKKRRQRRRPPQ-*βA*-GYRYKR**W**P | 124 |
| 32 | A14P43 | GRKKRRQRRRPPQ-*βA*-RKR**W**RYDP | 107 |
| 35 | A14P46 | GRKKRRQRRRPPQ-*βA*-RKRSRY**W**P | 72 |
| 43 | A14P59 | GRKKRRQRRRPPQ-*βA*-VKKRRL**W**P | 140 |
| 64 | CIGB-300 | cycle (Cys15-Cys 25) GRKKRRQRRRPPQ- βA-CWMSPRHLGTC | 64 |

To corroborate that the biological activity of the polypeptide A14P46 (SEQ ID NO: 35) is only attributed to the charge position (RKRSRYWP), a similar peptide without the Tat and βA sequence was synthesized using the synthesis procedure described in this example. The peptide D14P165, identified as SEQ ID NO: 45, was obtained with 95% purity and the analysis by ESI-MS corroborated its MW of 1146,7 Da. The phosphorylation inhibition assay was performed as described in Example 2 and 85% inhibition was obtained, confirming the ability of the peptide to inhibit CK2-mediated phosphorylation of HPV-16 E7 protein.

Taking into account that the polypeptide A14P46 showed biological activity results similar to CIGB-300 in the NCI-H125 cell line, the behavior of such polypeptide in the pancreatic adenocarcinoma tumor line (AsPC-1) was studied, compared to the CIGB-300. The cell proliferation assay was performed following the procedure previously described (Perera Y, et al., 2012, J. Pept. Sci, 18 (4):215-23). As shown in Figure 6, both molecules are capable of producing a dose-dependent effect on the proliferation of AsPC-1 cells. IC₅₀ values of 125 µM for A14P46 and 102 µM for CIGB-300 were obtained, which show that both peptides have similar potency in this cell line.

It is shown that the polypeptide A14P46 (SEQ ID NO: 35) is comparable to CIGB-300 in its ability to inhibit cell proliferation in the NCI-H125 and AsPC-1 cell lines (Figures 5 and 6), however it is superior because is a linear polypeptide of smaller size, chemically more stable, not including either methionine or cysteine in its sequence, which makes it more attractive from a productive point of view.

### Example 5. Optimization of the polypeptide identified as SEQ ID NO: 35 by introducing a second Trp residue in its sequence.

Based on the results obtained in Example 4, another Trp residue was introduced at positions X₁, X₄ and X₅ of the A14P46 polypeptide, and its impact on biological activity was evaluated. Polypeptides were synthesized on MBHA resin using Fmoc/tBu chemistry, (Field GB y Noble RL. Int. J. Peptide Protein Res. 1990; 35(3):161-214), purified by RP-HPLC, and identity confirmed by ESI-MS. As shown in Table 6, the three peptides were obtained with more than 95% purity and ESI-MS analysis confirmed the identity of the molecule.

**Table 6. Characterization of the peptides generated for the optimization of the peptide identified as SEQ ID NO: 35.**

| **SEQ ID NO:** | **Code** | **Sequence** | **Purity (%)** | **MW (Da)** |
|---|---|---|---|---|
| 46 | A15P35 | GRKKRRQRRRPPQ-βA- WKRSRYWP | 99.7 | 2947.7 |
| 47 | A15P36 | GRKKRRQRRRPPQ-βA- RKRWRYWP | 95.1 | 3016.8 |
| 48 | A15P37 | GRKKRRQRRRPPQ-βA- RKRSWYWP | 95.1 | 2947.7 |

Bold highlights the introduction of the second Trp residue in the peptide sequence identified as SEQ ID NO: 35.

The evaluation of the antiproliferative activity of synthetic polypeptides A15P35, A15P36 and A15P37 (SEQ ID NO: 46 - SEQ ID NO: 48) was performed on the NCI-H125, AsPC-1, PanC-1 (pancreatic carcinoma) and OCI-AML3 (acute myeloid leukemia-3) cell lines. The test was carried out according to the procedure previously described (Perera Y, et al., 2012, J. Pept. Sci, 18 (4):215-23). Figure 7 shows the dose-effect curves of the three polypeptides in the NCI-H125 cell line, in comparison with its precursor A14P46 and the control peptide CIGB-300. The polypeptides A15P35, A15P36 and A15P37 were able to produce a dose-dependent effect on the proliferation of NCI-H125 cells. In the AsPC-1, PanC-1 and OCI-AML3 lines, the three polypeptides also showed a dose-response pattern.

Table 7 summarizes the IC₅₀ values of the A15P35, A15P36 and A15P37 polypeptides obtained in the NCI-H125, AsPC-1, PanC-1 and OCI-AML3 cell lines, in comparison with their precursor A14P46 and with the control peptide. CIGB-300. The IC₅₀ values of the polypeptide A15P35 are similar to those obtained for its precursor A14P46 and for CIGB-300, in the four lines studied, which shows that the introduction of Trp in position X1 of the sequence is irrelevant. In contrast, the IC₅₀ values obtained for polypeptides A15P36 and A15P37 were lower than those obtained for polypeptide A14P46 and peptide CIGB-300, especially in pancreatic cancer cell lines (AsPC-1, PanC-1). and acute myeloid leukemia (OCI-AML3).

**Table 7. Values of IC₅₀ for the polypeptides identified as SEQ ID NO: 46 - SEQ ID NO: 48 in the cell proliferation assay with the NCI-H125, AsPC-1, PanC-1 and OCI-AML3 lines.**

| **SEQ ID NO:** | **Code** | **IC₅₀ (µM)** | | | |
|---|---|---|---|---|---|
| | | **NCl-H125** | **AsPC-1** | **PanC-1** | **OCI-AML3** |
| 46 | A15P35 | 106 | 127 | 145 | 130 |
| 47 | A15P36 | 42 | 26 | 54 | 9 |
| 48 | A15P37 | 46 | 23 | 65 | 21 |
| 35 | A14P46 | 72 | 125 | 166 | 70 |
| 64 | CIGB-300 | 64 | 102 | 156 | 63 |

The values obtained for the control peptides A14P46 and CIGB-300 are included.

To corroborate that the biological activity of the A15P36 polypeptides (SEQ ID NO: 47) and A15P37 (SEQ ID NO: 48) it is only attributed to the charge RKRWRYWP and RKRSWYWP, both peptides were synthesized without the Tat or βA sequence, using the synthesis procedure described in this example. The peptides D15P105 (SEQ ID NO: 49) and D15P106 (SEQ ID NO: 50) they were obtained with more than 95% purity, and the ESI-MS analysis corroborated their molecular masses of 1245.7 Da and 1176.6 Da, respectively. The phosphorylation assay was performed as described in Example 2, and phosphorylation inhibition values of 87% and 82%, respectively, were obtained, confirming the ability of both peptides to inhibit CK2-mediated phosphorylation in protein HPV-16 E7.

The results of this example show that the introduction of a second Trp in positions X4 and X5 of the polypeptide sequence A14P46 (SEQ ID NO: 35), favors the interaction of the peptide with the phosphoacceptor site, and therefore its ability to inhibit phosphorylation by CK2. A15P36 and A15P37 polypeptides are shown to be more potent than CIGB-300 in their ability to inhibit cell proliferation in pancreatic cancer (AsPC-1, PanC-1) and acute myeloid leukemia (OCI-AML3) cell lines. Both polypeptides have the additional advantage that they are linear sequences of smaller size, chemically more stable, since they do not have sensitive residues such as methionine and cysteine. In addition, they are more attractive from the productive point of view.

### Example 6. Optimization of the polypeptides identified as SEQ ID NO: 47 and SEQ ID NO: 48 by introducing unnatural amino acids (D- and L-N-methyl amino acids).

In order to protect polypeptides A15P36 (SEQ ID NO: 47) and A15P37 (SEQ ID NO: 48) from the action of endopeptidases in biological fluids, non-natural amino acids were introduced in those sites of the sequence that were more prone to degradation by trypsin and chymotrypsin. The sites most sensitive to proteolysis were predicted with the help of the Expasy site (http://www.expasy.org) (Gasteiger E., et al., 2003, Nucleic Acids Research. 31(13): 3784-3788). D-Arg was incorporated into R8 of Tat and the protection of K16, R17 and Y20 was evaluated with the introduction of D-Lys, D-Arg and NMe-Tyr, respectively. The N-terminus of each polypeptide was acetylated to protect it from N-peptidases. Polypeptides E17P01, E17P02 and E17P03 (SEQ ID NO: 51 - SEQ ID NO: 53) are derivatives of A15P36 and polypeptides D17P78, D17P79 and D17P80 (SEQ ID NO: 54 - SEQ ID NO: 56) are derivatives of A15P37. Polypeptides were synthesized on MBHA resin using Fmoc/tBu chemistry, purified by RP-HPLC, and identity confirmed by ESI-MS. As shown in Table 8, all were obtained with more than 95% purity and ESI-MS analysis confirmed the identity of the molecule.

**Table 8. Characterization of the polypeptides identified as SEQ ID NO: 51- SEQ ID NO: 56.**

| **SEQ ID NO:** | **Code** | **Sequence** | **Purity (%)** | **MW (Da)** |
|---|---|---|---|---|
| 51 | E17P01 | Ac-GRKKRRQ(D-Arg)RRPPQ- βA-R(D-Lys) RWRYWP | 98.4 | 3058.8 |
| 52 | E17P02 | Ac-GRKKRRQ(D-Arg)RRPPQ- βA-RK(D-Arg)WRYWP | 96.3 | 3058.8 |
| 53 | E17P03 | Ac-GRKKRRQ(D-Arg)RRPPQ- βA-RKRWR(NMe-Tyr)WP | 96.6 | 3072.8 |
| 54 | D17P78 | Ac-GRKKRRQ(D-Arg)RRPPQ- βA-R(D-Lys)RSWYWP | 97.3 | 2989.7 |
| 55 | D17P79 | Ac-GRKKRRQ(D-Arg)RRPPQ- βA-RK(D-Arg)SWYWP | 98.2 | 2989.7 |
| 56 | D17P80 | Ac-GRKKRRQ(D-Arg)RRPPQ- βA-RKRSW(NMe-Tyr)WP | 95.7 | 3003.7 |

To corroborate that the introduction of unnatural amino acids does not affect the ability of the polypeptides to interact with the phosphoacceptor site of the CK2 enzyme in the E7 protein, the procedure described in Example 1 was followed. The six resin-coupled polypeptides developed a deep blue color after enzyme assay, evidencing interaction with the phosphoacceptor site of the CK2 enzyme on the model E7 substrate. On the other hand, to demonstrate that the modification does not affect the effect of the peptides on the phosphorylation by the enzyme CK2 in the E7 protein, the procedure described in Example 2 was followed. The polypeptides E17P01-E17P03 and D17P78-D17P80, identified with SEQ ID NO: 51 - SEQ ID NO: 56, maintain the ability to inhibit CK2-mediated phosphorylation in the E7 protein of HPV-16, phosphorylation inhibition values between 83 and 90% were obtained.

Antiproliferative assays were performed on the NCI-H125, Hep2C (laryngeal carcinoma) cell lines, and on two bladder cancer lines (MGH-U3 and MGH-U4). The test was carried out according to the procedure previously described (Perera Y., et al, 2012, Pept. Sci, 18 (4):215-23).

Figures 8 and 9 show the dose-effect curves in the NCI-H125 cell line of the polypeptides E17P01, E17P02 and E17P03 (SEQ ID NO: 51- SEQ ID NO: 53) and D17P78, D17P79 and D17P80 (SEQ ID NO: 54- SEQ ID NO: 56), respectively. The control CIGB-300 peptide and the precursor polypeptides A15P36 and A15P37, were included respectively. The six polypeptides were able to produce a dose-dependent effect on the proliferation of cells of the NCI-H125 line. In the Hep2C, MGH-U3 and MGH-U4 cell lines all six polypeptides also showed a dose-response pattern.

Table 9 summarizes the IC₅₀ values of the polypeptides E17P01, E17P02, E17P03, D17P78, D17P79 and D17P80 (SEQ ID NO: 51- SEQ ID NO: 56) obtained in the cell lines NCI-H125, Hep2C, MGH- U3 and MGH-U4, in comparison with their precursors and the control peptide CIGB-300.

Table 9 shows that the IC₅₀ values of the polypeptides identified as SEQ ID NO: 51 - SEQ ID NO: 56, in the NCI-H125, Hep2C, MGH-U3 and MGH-U4 cell lines, are lower than those obtained for the peptide CIGB-300, which shows that they are more powerful in terms of their antiproliferative effect. Surprisingly, in the case of the bladder cancer lines (MGH-U3 and MGH-U4), the IC₅₀ values are one order of magnitude lower than the CIGB-300, which is very attractive, as it is a little explored niche, unresolved and with a high impact on health. Modifications to the A15P36 and A15P37 polypeptides were introduced to increase stability to proteases. Surprisingly, however, the ability to inhibit CK2 phosphorylation was also enhanced.

**Table 9. Values of the IC₅₀ for the polypeptides identified as SEQ ID NO: 51 - SEQ ID NO: 56 in the NCI-H125, Hep2C, MGH-U3 and MGH-U4 cell lines.**

| **SEQ ID NO:** | **Code** | **IC₅₀ (µM)** | | | |
|---|---|---|---|---|---|
| | | **NCl-H125** | **Hep2C** | **MGH-U3** | **MGH-U4** |
| 51 | E17P01 | 20 | 11 | 65 | 53 |
| 52 | E17P02 | 27 | 26 | 55 | 38 |
| 53 | E17P03 | 47 | 11 | 29 | 33 |
| 54 | D17P78 | 14 | 20 | 86 | 89 |
| 55 | D17P79 | 21 | 18 | 77 | 69 |
| 56 | D17P80 | 13 | 24 | 65 | 70 |
| 47 | A15P36 | 42 | 44 | - | - |
| 48 | A15P37 | 46 | 48 | - | - |
| 64 | CIGB-300 | 64 | 61 | 327 | 335 |

The values obtained for the control polypeptides A15P36, A15P37 and CIGB-300 are included.

To corroborate that the biological activity of the peptides identified as SEQ ID NO: 51 - SEQ ID NO: 56 is only attributed to their charges, peptides without the Tat and βA sequence were synthesized, using the synthesis procedure described in this same example. Peptides D17P81-D17P86 (SEQ ID NO: 57 - SEQ ID NO: 62) were obtained with more than 95% purity and ESI-MS analysis confirmed the identity of the molecules obtained with the designed ones (Table 10).

**Table 10. Characterization of the peptides identified as SEQ ID NO: 57- SEQ ID NO: 62.**

| **SEQ ID NO:** | **Code** | **Sequence** | **Purity (%)** | **MW (Da)** |
|---|---|---|---|---|
| 57 | D17P81 | R(D-Lys) RWRYWP | 98.5 | 1245.7 |
| 58 | D17P82 | RK(D-Arg)WRYWP | 97.6 | 1245.7 |
| 59 | D17P83 | RKRWR(NMe-Tyr)WP | 95.8 | 1259.7 |
| 60 | D17P84 | R(D-Lys)RSWYWP | 96.3 | 1176.7 |
| 61 | D17P85 | RK(D-Arg)SWYWP | 97.8 | 1176.7 |
| 62 | D17P86 | RKRSW(NMe-Tyr)WP | 95.5 | 1190.6 |

The phosphorylation assay was performed as described in Example 2, and phosphorylation inhibition values between 83 and 90% were obtained, confirming the ability of these peptides to inhibit CK2-mediated phosphorylation of the HPV-16 E7 protein.

In summary, the polypeptides identified as SEQ ID NO: 51 - SEQ ID NO: 56 are more potent than the peptide CIGB-300, in terms of their ability to inhibit cell proliferation, in particular their inhibitory activity in lines of bladder cancer (MGH-U3 and MGH-U4). In addition, these peptides are linear, smaller in size, and chemically more stable, which brings undoubted advantages from the production point of view.

### Example 7. Antitumor effect of a polypeptide inhibitor of CK2 phosphorylation in a human bladder tumor model implanted in athymic mice.

For these tests, athymic female BalbC mice, between 6 and 8 weeks old, were used. MGH-U3 cells, from bladder cell carcinoma, were used. For tumor implantation in this model, cells were suspended in PBS at a concentration of 1 000 000 cells per milliliter. The cell suspension was inoculated subcutaneously in the abdominal cavity of the animals. Administration of E17P01 polypeptide (identified as SEQ ID NO: 51) or CIGB-300 was started 5 days after the tumor cells were inoculated, and continued for 5 consecutive days, intraperitoneally. The polypeptide and peptide were dissolved in PBS. The administered dose was 20 µg, which corresponds to 1 mg/kg of weight. At different times, the tumor volume (in mm³) was measured, as a parameter to evaluate the antitumor effect of the polypeptide. As seen in Figure 10, the E17P01 polypeptide showed its ability to inhibit tumor progression, which was greater than that observed for CIGB-300. These results evidenced the antitumor efficacy of the polypeptide identified as SEQ ID NO: 51 in a model of human tumors implanted in experimental animals.

## Claims

1. Linear peptide that inhibits the phosphorylation mediated by the enzyme Casein Kinase 2 (CK2) **characterized by** an amino acid sequence selected from the group consisting of the sequences identified as SEQ ID NO: 45, SEQ ID NO: 49, SEQ ID NO: 50, and SEQ ID NO: 57 - SEQ ID NO: 62.

2. Polypeptide comprising the linear peptide that inhibits the phosphorylation mediated by the enzyme Casein Kinase 2 (CK2) of claim 1 and an intracellular penetrating peptide.

3. The polypeptide of claim 2 wherein the intracellular penetrating peptide is a peptide from the Tat1 protein of the Human Immunodeficiency Virus.

4. The polypeptide of claim 2 wherein the intracellularly penetrating peptide has an amino acid sequence that is identified as SEQ ID NO: 65 or SEQ ID NO: 66.

5. The polypeptide of claim 4 that has an amino acid sequence selected from the group consisting of the sequences identified as SEQ ID NO: 35, SEQ ID NO: 47, SEQ ID NO: 48, and SEQ ID NO: 51 - SEQ ID NO: 56.

6. A pharmaceutical composition comprising at least one linear peptide that inhibits Casein Kinase 2 (CK2)-mediated phosphorylation of claim 1 or a polypeptide of any of claims 2-5 and a pharmaceutically acceptable excipient.

7. The pharmaceutical composition of claim 6 that is formulated for its administration by systemic, intratumoral, oral or mucosal route.

8. The pharmaceutical composition of claim 6 which is useful for the treatment of solid or liquid tumors.

9. Use of the linear peptide that inhibits phosphorylation mediated by Casein Kinase 2 (CK2) of claim 1 or of the polypeptide of any of claims 2-5 for the manufacture of a medicament.

10. The use according to claim 9 where the medicament is used in the treatment of solid or liquid tumors.

11. A method of treatment of solid or liquid tumors in an individual in need thereof comprising administering a therapeutically effective amount of the pharmaceutical composition of claim 6.

12. The method of treatment of claim 11 wherein the solid tumor is of the lung, cervix, larynx, pancreas, or bladder.

13. The method of treatment of claim 11 wherein the liquid tumor is chronic lymphocytic leukemia, T-type acute lymphoblastic leukemia or acute myeloid leukemia.
